# EUROPÄISCHE PATENTANMELDUNG

(11) **EP 1 847 318 A2**
(43) Veröffentlichungstag der Anmeldung: **24.10.2007**
(21) Anmeldenummer: 07003000.2
(22) Anmeldetag: 13.02.2007
(51) Int. Cl.: B01J 20/28, B01J 20/20

(54) **Mit Adsorbentien beaufschlagte Plattenmaterialien für den Trockenbau**

(30) Priorität: 18.04.2006 DE 102006018097; 12.05.2006 DE 102006022178
(71) Anmelder: Blücher GmbH, 40699 Erkrath (DE)
(72) Erfinder: von Blücher, Hasso, 40699 Erkrath (DE)
(74) Vertreter: Gesthuysen, von Rohr & Eggert

(57) **Zusammenfassung**

Gegenstand der Erfindung ist ein Baumaterial für den Innenausbau auf Basis eines plattenförmigen Werkstoffs für den Trockenbau, insbesondere einer Bauplatte, wobei der plattenförmige Werkstoff Aktivkohle aufweist. Das Baumaterial eignet sich in hervorragender Weise zur Beseitigung von Emissionen von Schad- und/oder Geruchsstoffen mittels Adsorption, vorzugsweise in Gebäuden, insbesondere zu Zwecken der Entfernung von Schad- und/oder Geruchsstoffen in schad- und/oder geruchsstoffbelasteten Räume, zu Zwecken der Sanierung schad- und/oder Geruchsstoffbelasteter Räume und/oder zu Zwecken der Raumluftverbesserung.

## Beschreibung

Die vorliegende Erfindung betrifft mit Adsorbentien beaufschlage Plattenmaterialien für den Trockenbau sowie deren Verwendung. Insbesondere betrifft die vorliegende Erfindung ein Baumaterial für den Innenausbau auf Basis eines plattenförmigen Werkstoffs für den Trockenbau nach dem Oberbegriff von Anspruch 1 sowie dessen Verwendung.

Aufgrund eines steigenden Umweltbewußtseins einerseits, aber auch infolge hochempfindlicher Analysenmethoden andererseits rückt die Belastung unserer Umgebung bzw. Umwelt durch Schadstoffe immer mehr ins Licht der Öffentlichkeit. So besteht beispielsweise für den Bereich schad- und/oder geruchsstoffbelasteter Gebäude ein gesteigerter Bedarf, diese mit möglichst wenig Aufwand von Schad- und/oder Geruchsstoffen zu befreien und erforderlichenfalls zu sanieren. Weiterhin besteht ein Bedarf, das Raumklima bzw. die Raumluft in schad- und/oder geruchsstoffbelasteten Räumen entsprechend zu verbessern.

Im Rahmen der vorliegenden Erfindung werden als Schadstoffe insbesondere solche Stoffe bezeichnet, die bereits in geringer Menge beim Menschen Irritationen, Allergien oder Krankheiten auslösen können. Hierzu zählen beispielsweise Holzschutzmittel, wie Pentachlorphenol (PCP) und Lindan, Weichmacher, wie polychlorierte Biphenyle (PCB), oder aber auch Formaldehyd. So wurde beispielsweise Formaldehyd in Spanplatten eingesetzt und ist mittlerweile als karzinogen eingestuft. Auch können Kohlenwasserstoffe, die gegebenenfalls aromatisch sind, bzw. deren chlorierte Derivate als Schadstoffe im Sinne der vorliegenden Erfindung beispielsweise aus Lacken, Anstrichen, Klebstoffen oder dergleichen entweichen.

Besonders problematische Schadstoffe sind insbesondere die vorgenannten PCB, die beispielsweise als Weichmacher in Fugendichtungsmassen, insbesondere bei der Errichtung von Bauten aus Fertigelementen, eingesetzt wurden. Neuere Erkenntnisse haben gezeigt, daß PCB im Laufe der Zeit aus Dichtungsmassen sowohl in die angrenzenden Betonelemente diffundieren als auch von den Fugendichtungen in die Umgebungsluft abgegeben werden können.

Über den Luftaustausch wird dann die mit PCB belastete Luft über das gesamte Gebäude verteilt. Das so von den Fugenabdichtungen freigesetzte PCB schlägt sich in den Räumen teilweise partikelgebunden nieder, löst sich zu einem großen Teil aber auch in Wandfarben und Kunststoffen, was dazu führt, daß nach einiger Zeit der Freisetzung eine Reihe von sogenannten sekundären Emissionsquellen gebildet werden, wobei hier insbesondere gestrichene Wand- und Deckenflächen zu nennen sind. Diese Sekundärquellen enthalten dann im allgemeinen eine derart große Menge an PCB, daß sie eine große Emissionsfläche darstellen, so daß ein alleiniges Entfernen der Fugenabdichtungen kein Absinken der PCB-Konzentrationen in der Raumluft unter die vorgeschriebenen Werte bewirken kann.

Eine weitere Quelle von Schad- und/oder Geruchsstoffen können beispielsweise Teppichböden darstellen. Die Emissionen entstehen z. B. dadurch, daß Ausgangsstoffe dieser Produkte unter dem Einfluß von Feuchtigkeit und/oder unter dem Einfluß von Bestandteilen der Untergrundmaterialien reagieren, so daß der Boden selbst nach dem Entfernen des Bodenbelags weiter Geruchs- und/oder Schadstoffe emittiert, so daß entweder der gesamte Fußboden entfernt oder aber ein Zwischenboden mit Hinterlüftung verlegt werden muß.

Eine weitere Emissionsquelle für unangenehme, manchmal auch gesundheitsschädliche Emissionen sind Zusätze zum Baumaterial selbst. Beispielsweise sind viele Gebäude von Ammoniakausdünstungen betroffen, die auf die Verwendung von Ammoniumsalzen, Harnstoffen oder organischen Aminen im weitesten Sinne als Frostschutzmittel für Beton und Mörtel zurückzuführen sind. Des weiteren ist als Ursache für die Ausdünstung von Aminen bisweilen auch eine vormalige Nutzung eines Raumes z. B. zu Zwecken der Tierhaltung zu nennen, welche dazu führt, daß Bauelemente über einen längeren Zeitraum mit den Schadstoffen aus der Luft beaufschlagt werden. Selbst bei einem Entfernen der Primäremissionsquellen dieser Substanzen, etwa bei einer Umwidmung eines Stallgebäudes zu einem Wohn- oder Geschäftsraum, werden dann diese Schad- und/oder Geruchsstoffe von den Sekundärquellen, nämlich Wand- und Deckenflächen, weiter emittiert.

Eine Möglichkeit zur Entfernung von PCB emittierenden Schadstoffquellen in Form von PCB belasteten Fugendichtmassen ist in der DE 40 28 434 A1 vorgesehen. Hierbei wird durch geeignete Maßnahmen die Dichtungsmasse, d. h. nur die Primärquelle, herausgeschnitten und entsorgt. Das Verfahren greift damit aber lediglich auf die Primärquellen zu, so daß eine Dekontaminierung der Sekundärquellen unberücksichtigt bleibt.

Weiter ist aus der DE 38 18 993 A1 ein Verfahren zur Sanierung schadstoffbelasteter Räume bekannt, bei dem die schadstoffbelastete Raumluft dadurch gereinigt wird, daß durch geeignete Maßnahmen Luft an Adsorbentien künstlich oder nur durch Eigenzirkulation vorbeigeführt wird. Beispielsweise wird die schadstoffbelastete Luft durch mit Adsorbentien beschickte Adsorptionstürme gepreßt. Eine andere dort beschriebene Möglichkeit besteht darin, die Luft an großflächigen, mit Adsorbentien beladenen Flächengebilden, wie beispielsweise Vorhängen, vorbeizuführen. Dieses Verfahren hat den entscheidenden Nachteil, lediglich auf die bereits belastete Raumluft zurückzugreifen, welche sich immer wieder mit der schadstoffbelasteten Luft vermischt, so daß allenfalls ein Verdünnungseffekt erzielt wird.

Weiterhin sind textile, mit Adsorbentien beaufschlagte, im allgemeinen mit einer wasserundurchlässigen, aber wasserdampfdurchlässigen Beschichtung versehene Flächematerialien bekannt, welche in Abhängigkeit von der Schadstoffquelle als Tapete, Bodenbelag oder dergleichen ausgebildet sind und über die entsprechenden Schad- und/oder Geruchsstoffe emittierenden Quellen aufgebracht werden (vgl. z. B. DE 44 32 834 A1, DE 44 47 844 C2, DE 196 07 423 A1 und DE 200 08 162 U1). Die dort beschriebenen Materialien eignen sich aber nicht für den Trockenbau, insbesondere nicht als Wand-, Boden- oder Deckenelemente.

Schließlich sind aus der WO 2005/026465 A2 Baumaterialien für den Innenausbau auf Basis von Gipsbauplatten, wie beispielsweise Gipskartonplatten und Gipsfaserplatten, bekannt, welche Zeolithe in Mengen von 1 bis 25 Gew.-% enthalten, und dazu bestimmt sind, Luftschadstoffe in Innenräumen, wie Formaldehyde, Ammoniak, Tabakrauch etc., zu reduzieren. Die dort eingesetzten Zeolithe liefern aber nicht immer das gewünschte Ergebnis, insbesondere aufgrund ihres nicht immer ausreichenden Vermögens, Schad- und/oder Geruchsstoffe aus der Umgebungsluft abzubauen oder zu sorbieren. Da die Zeolithe bei der Herstellung der Gipsplatten in die Gipsmasse eingerührt werden, ist zudem ein großer Teil ihrer Oberfläche nicht für die aufzunehmenden bzw. zu zersetzenden Schad- und Geruchsstoffe frei zugänglich, so daß die Schad- und Geruchsstoffe durch die Gipsmasse hindurch zu den hierin eingelagerten Zeolithen diffundieren müssen. Aus den vorgenannten Gründen müssen relativ große Mengen an Zeolithen eingesetzt werden, was aber die Baumaterialien als solche, insbesondere im Hinblick auf ihren Verbund oder ihre Stabilität, beeinträchtigen kann.

Das der vorliegenden Erfindung zugrundeliegende Problem liegt daher insbesondere in der Bereitstellung von Baumaterialien für den Innenausbau, vorzugsweise auf Basis plattenförmiger Werkstoffe für den Trockenbau, welche die zuvor geschilderten Nachteile des Standes der Technik zumindest weitgehend vermeiden oder aber wenigstens abschwächen.

Insbesondere sollen Baumaterialien bzw. Bauplatten mit verbesserten Eigenschaften bereitgestellt werden. Dabei sollen insbesondere für den Innenausbau geeignete Baumaterialien, vorzugsweise in Form von sogenannten Bauplatten, bereitgestellt werden, mit denen sich Geruchs- und/oder Schadstoffe aus der Umgebungsluft effizient reduzieren lassen.

Zur Lösung der vorgenannten Aufgabenstellung wird ein Baumaterial nach Anspruch 1 bzw. dessen Verwendung nach Anspruch 17 vorgeschlagen. Weitere, vorteilhafte Ausgestaltungen sind Gegenstand der betreffenden Unteransprüche.

Die Anmelderin hat nun überraschenderweise herausgefunden, daß das zuvor geschilderte Problem dadurch gelöst werden kann, daß man Baumaterialien für den Innenausbau, insbesondere auf Basis sogenannter Bauplatten, mit Aktivkohle versieht bzw. beaufschlagt.

Gegenstand der vorliegenden Erfindung - gemäß einem **ersten** Aspekt der vorliegenden Erfindung - ist somit ein Baumaterial für den Innenausbau auf Basis eines plattenförmigen Werkstoffs für den Trockenbau, wobei der plattenförmige Werkstoff Aktivkohle aufweist bzw. hiermit versehen und/oder beaufschlagt ist.

Als plattenförmige Werkstoffe im Sinne der vorliegenden Erfindung können insbesondere sogenannte Bauplatten, vorzugsweise auf Basis von Gips, aber auch auf Basis anderer Materialien, eingesetzt werden. Hierbei kann es sich beispielsweise um Bauplatten aus Gips ("Gipsbauplatten"), Gipskartonbauplatten (z. B. Rigips^{®}-Platten), Bauplatten aus Gipsfaserverbundstoffen (z. B. Gipsfaserplatten), Bauplatten aus Zementfaserverbundstoffen (z. B. Faserzementplatten, wie Eternit^{®}-Platten), Bauplatten aus Glasfasern, Bauplatten aus Kohlenstoffasern, Holzbauplatten, Holzfaserbauplatten, Kunststoffbauplatten oder dergleichen sowie Bauplatten aus Kombinationen der vorgenannten Materialien handeln. Erfindungsgemäß bevorzugt sind Gipsbauplatten oder aber Gipskartonbauplatten, insbesondere Gipskartonbauplatten mit ein- oder beidseitiger Kartonageaußenbeschichtung und einer Gipskernschicht.

Erfindungsgemäß eingesetzte Bauplatten bzw. plattenförmige Werkstoffe haben im allgemeinen Dicken von 0,1 bis 20 cm, insbesondere 0,5 bis 10 cm, vorzugsweise 1 bis 10 cm.

Die Befestigung der Aktivkohle in und/oder an den plattenförmigen Werkstoffen bzw. Bauplatten kann in vielfältigster Art und Weise erfolgen. Dies ist dem Fachmann an sich geläufig.

Beispielsweise kann die Aktivkohle in den plattenförmigen Werkstoffen bei deren Herstellung eingearbeitet sein (z. B. im Falle von Gips- oder Gipskartonplatten in den Gips eingerührt werden); diese Ausführungsform ist erfindungsgemäß jedoch weniger bevorzugt, da ein Teil der aktiven Oberfläche der Aktivkohle dann mit dem Material der Bauplatte bzw. des Werkstoffs belegt ist und nicht mehr für Adsorptionszwecke zur Verfügung steht.

Erfindungsgemäß bevorzugt ist es, wenn der plattenförmige Werkstoff bzw. die Bauplatte an mindestens einer seiner bzw. ihrer beiden Oberflächen mit Aktivkohle versehen und/oder beaufschlagt ist. In erfindungsgemäß bevorzugter Weise ist somit die Aktivkohle nur an der bzw. den Oberfläche(n) des plattenförmigen Werkstoffs fixiert. Dies kann beispielsweise dadurch erfolgen, daß ein Klebstoff auf dem plattenförmigen Werkstoff aufgetragen wird, wobei ein punktförmiger und/oder punktrasterförmiger Klebstoffauftrag bevorzugt ist, und nachfolgend die Aktivkohle hieran zum Haften gebracht wird. Alternativ kann die Aktivkohle aber auch in die Oberfläche(n) des plattenförmigen Werkstoffs bzw. der Bauplatte bei dessen bzw. deren Herstellung eingearbeitet sein, beispielsweise durch Eindrücken in den noch feuchten Gips oder Zementfaserverbundstoff einer entsprechenden Werkstoff- bzw. Bauplatte.

Die Fixierung der Aktivkohle an dem plattenförmigen Werkstoff bzw. seine Einarbeitung hieran und/oder hierin erfolgt insbesondere derart, daß mindestens 25 %, insbesondere mindestens 30 %, vorzugsweise mindestens 40 %, besonders bevorzugt mindestens 50 %, der Oberfläche der Aktivkohle für zu adsorbierende Schad- und/oder Geruchsstoffe frei zugänglich ist. Wird beispielsweise die Aktivkohle (z. B. in Form von Aktivkohlekörnern, vorzugsweise Aktivkohlekügelchen) mittels eines Klebstoffs an einer oder aber an beiden Oberflächen des plattenförmigen Werkstoffs fixiert, wird vorteilhafterweise die Aktivkohle nicht vollständig in den Klebstoff eingedrückt, so daß sie noch freie Oberfläche in dem vorgenannten Maße aufweist. Entsprechendes gilt beim Einarbeiten in den plattenförmigen Werkstoff bei dessen Herstellung (z. B. bei Gipsplatten durch Eindrücken der Aktivkohle in den noch feuchten Gips). Freie Oberfläche der Aktivkohle bzw. für die zu adsorbierenden Schad-/Geruchsstoffe frei zugängliche Oberfläche der Oberfläche in diesem Zusammenhang bezeichnet die aktive, für Adsorptionsvorgänge verfügbare Oberfläche der Aktivkohle, welche im allgemeinen der Gesamtporenoberfläche der Aktivkohle entspricht. Dies bedeutet, daß im allgemeinen die Fixierung der Aktivkohle an dem plattenförmigen Werkstoff bzw. seine Einarbeitung hieran und/oder hierin insbesondere derart erfolgt, daß mindestens 25 %, insbesondere mindestens 30 %, vorzugsweise mindestens 40 %, besonders bevorzugt mindestens 50 %, der Gesamtporenoberfläche der Aktivkohle für zu adsorbierende Schad- und/oder Geruchsstoffe frei zugänglich ist

Gemäß einer alternativen Ausführungsform kann die Aktivkohle aber auch an einem vorzugsweise flächigen Träger, insbesondere einem textilen Flächengebilde, wie beispielsweise einem Papier, einem Karton, einer Pappe oder dergleichen, fixiert sein, wobei dieser mit Aktivkohle beaufschlagte Träger dann mit dem plattenförmigen Werkstoff verbunden ist, insbesondere mittels Laminierung, Verkleben oder dergleichen.

Die Menge des plattenförmigen Werkstoffs an Aktivkohle ist derart ausgelegt, daß die durch die Aktivkohle bereitgestellte Adsorptionskapazität ausreicht, um bei der Verwendung des plattenförmigen Werkstoffs aus der Umgebung stammende Emissionen von Schad- und/oder Geruchsstoffen dauerhaft adsorptiv zu binden.

Die Menge an Aktivkohle kann in weiten Bereichen variieren. Insbesondere enthält der plattenförmige Werkstoff bzw. die Bauplatte nach der vorliegenden Erfindung Aktivkohle in einer Menge, insbesondere Beladungs- und/oder Auflagemenge, von insgesamt 20 bis 750 g/m², insbesondere 25 bis 500 g/m², vorzugsweise 30 bis 400 g/m², besonders bevorzugt 50 bis 300 g/m²,

Was die Aktivkohle als solche anbelangt, so kann diese in beliebigen Erscheinungsformen eingesetzt werden.

Beispielsweise kann die Aktivkohle entsprechend einer erfindungsgemäß weniger bevorzugten Ausführungsform pulverförmig ausgebildet sein.

Erfindungsgemäß bevorzugt ist es jedoch, wenn die Aktivkohle kornförmig ("Komkohle"), vorzugsweise kugelförmig ("Kugelkohle"), ausgebildet ist. Dabei weisen die Aktivkohlekörner, vorzugsweise Aktivkohlekugeln, vorteilhafterweise mittlere Teilchendurchmesser im Bereich von 0,01 bis 2,0 mm, insbesondere 0,05 bis 1,0 mm, vorzugsweise 0,1 bis 1,0 mm, auf. Bei dieser Ausführungsform wird die Beladungs- und/oder Auflagemenge vorteilhafterweise derart bemessen, daß die einzelnen Aktivkohlekörner, vorzugsweise Aktivkohlekugeln, einen mittleren Abstand zueinander auf dem plattenförmigen Werkstoff aufweisen, der mindestens der Hälfte, insbesondere mindestens Dreiviertel, vorzugsweise mindestens dem Einfachen, besonders bevorzugt mindestens dem Eineinhalbfachen, des mittleren Teilchendurchmessers der Aktivkohlekörner, vorzugsweise Aktivkohlekugeln, entspricht.

Erfindungsgemäß bevorzugt eingesetzte Aktivkohlekörner, vorzugsweise Aktivkohlekügelchen, weisen einen Berstdruck von mindestens 5 Newton, insbesondere einen Berstdruck im Bereich von 5 Newton bis 20 Newton, pro Aktivkohlekorn bzw. Aktivkohlekügelchen auf.

Gemäß einer alternativen Ausführungsform kann die Aktivkohle in Form von Aktivkohlefasern vorliegen. Gleichermaßen ist es möglich, eine Aktivkohle einzusetzen, welche eine Mischung aus faserförmiger und kornförmiger, vorzugsweise kugelförmiger, Aktivkohle umfaßt.

Um eine hohe Leistungsfähigkeit des erfindungsgemäßen Baumaterials zu gewährleisten, ist es vorteilhaft, wenn die eingesetzte Aktivkohle eine spezifische Oberfläche (BET) von mindestens 500 m²/g, insbesondere mindestens 750 m²/g, vorzugsweise mindestens 1.000 m²/g, besonders bevorzugt mindestens 1.200 m²/g, aufweist. Im allgemeinen weist die erfindungsgemäß eingesetzte Aktivkohle eine spezifische Oberfläche (BET) im Bereich von 500 bis 2.500 m²/g, insbesondere 750 bis 2.250 m²/g, vorzugsweise 900 bis 2.000 m²/g, besonders bevorzugt 1.000 bis 1.750 m²/g, auf.

Zur BET-Methode kann beispielsweise verwiesen werden auf Römpp Chemielexikon, 10. Auflage, Georg Thieme Verlag Stuttgart/New York, Stichwort: "BET-Methode", sowie auf die dort referierte Literatur, insbesondere Winnacker-Küchler, 3. Auflage, Band 7, Seiten 93 ff., sowie Z. Annal. Chem. 238, Seiten 187 bis 193 (1968).

Um eine hohe Effizienz des erfindungsgemäßen Baumaterials zu ermöglichen, ist es bevorzugt, wenn die eingesetzte Aktivkohle eine Aktivkohle mit einem Adsorptionsvolumen V_{ads} von mindestens 250 cm³/g, insbesondere mindestens 300 cm³/g, vorzugsweise mindestens 350 cm³/g, besonders bevorzugt mindestens 400 cm³/g, ist. Im allgemeinen wird eine Aktivkohle mit einem Adsorptionsvolumen V_{ads} von 250 bis 1.000 cm³/g, insbesondere 300 bis 900 cm³/g, vorzugsweise 350 bis 750 cm³/g, eingesetzt.

Erfindungsgemäß bevorzugt ist eine Aktivkohle mit einem Gesamtporenvolumen nach Gurvich von mindestens 0,50 cm³/g, insbesondere mindestens 0,55 cm³/g, vorzugsweise mindestens 0,60 cm³/g, besonders bevorzugt mindestens 0,65 cm³/g, ganz besonders bevorzugt mindestens 0,70 cm³/g. Im allgemeinen wird eine Aktivkohle mit einem Gesamtporenvolumen nach Gurvich von 0,50 bis 0,90 cm³/g, insbesondere 0,55 bis 0,85 cm³/g, vorzugsweise 0,60 bis 0,80 cm³/g, besonders bevorzugt 0,65 bis 0,80 cm³/g, ganz besonders bevorzugt 0,70 bis 0,75 cm³/g, eingesetzt.

Zu weiteren Einzelheiten bezüglich der Bestimmung des Gesamtporenvolumens nach Gurvich kann beispielsweise verwiesen werden auf L. Gurvich (1915), J. Phys. Chem. Soc. Russ. 47, 805, sowie auf S. Lowell et al., Characterization of Porous Solids and Powders: Surface Area Pore Size and Density, Kluwer Academic Publishers, Article Technology Series, Seiten 111 ff.

Die Anmelderin hat herausgefunden, daß als Aktivkohle insbesondere eine Aktivkohle mit großem Mikroporenvolumenanteil, bezogen auf das Gesamtporenvolumen der Aktivkohle, geeignet ist. Im Rahmen der vorliegenden Erfindung wird unter dem Begriff des Mikroporenvolumens insbesondere dasjenige Porenvolumen der Aktivkohle verstanden, welches durch Poren mit einem Durchmesser von ≤ 25 Å (2,5 nm), insbesondere ≤ 20 Å (2,0 nm), bereitgestellt wird.

Die Anmelderin hat nämlich überraschenderweise herausgefunden, daß die Reduzierung der Schad- und/oder Geruchsstoffkonzentrationen besonders effizient ist, wenn der Mikroporenvolumenanteil der eingesetzten Aktivkohle besonders hoch ist. Ohne sich auf eine bestimmte Theorie festlegen zu wollen, läßt sich die besonders gute Effizienz mit einer Aktivkohle mit besonders großem Mikroporenvolumenanteil darauf zurückführen, daß die Mikroporen aufgrund ihrer nur geringen Größe sozusagen von allen Seiten bzw. Wandungen mit den zu sorbierenden bzw. adsorbierenden Molekülen in Wechselwirkung treten können. Insbesondere wird eine Aktivkohle mit einem Anteil des Mikroporenvolumens, bezogen auf das Gesamtporenvolumen der Aktivkohle, von mindestens 60 %, insbesondere mindestens 65 %, bevorzugt mindestens 70 %, eingesetzt.

Insbesondere wird in erfindungsgemäß bevorzugter Weise eine Aktivkohle mit einem aus Poren mit Porendurchmessern von ≤ 25 Å, vorzugsweise ≤ 20 Å, gebildeten Mikroporenvolumenanteil von mindestens 60 %, insbesondere mindestens 65 %, bevorzugt mindestens 70 %, bezogen auf das Gesamtporenvolumen, eingesetzt.

Eine erfindungsgemäß bevorzugt eingesetzte Aktivkohle weist ein Mikroporenvolumen, d. h. ein aus Poren mit Porendurchmessern von ≤ 25 Å, vorzugsweise ≤ 20 Å, gebildetes Mikroporenvolumen, nach Carbon Black von mindestens 0,40 cm³/g, insbesondere mindestens 0,45 cm³/g, vorzugsweise mindestens 0,50 cm³/g, auf. Im allgemeinen liegt dieses Mikroporenvolumen nach Carbon Black im Bereich von 0,40 bis 0,80 cm³/g, insbesondere 0,45 bis 0,75 cm³/g, vorzugsweise 0,50 bis 0,60 cm³/g.

Zu weiteren Einzelheiten der Bestimmung der Porenoberfläche nach Carbon Black kann beispielsweise verwiesen werden auf R. W. Magee, Evaluation of the External Surface Area of Carbon Black by Nitrogene Adsorption, Presented at the Meeting of the Rubber Division of the American Chem. Soc., Oktober 1994, z. B. referiert in: Quantachrome Instruments, AUTOSORB-1 AS 1 WinVersion 1.50, Operating Manual, P/N 05061, Quantachrome Instruments 2004, Florida, USA, Seiten 71 ff.

Aufgrund der hohen Mikroporosität der erfindungsgemäß bevorzugt eingesetzten Aktivkohle weist diese gleichermaßen einen hohen spezifischen Mikroporenoberflächenanteil auf. Der spezifische Mikroporenoberflächenanteil, d. h. der Oberflächenanteil, der auf Poren mit Porendurchmessern von ≤ 25 Å, vorzugsweise ≤ 20 Å, zurückgeht beträgt mindestens 70 %, insbesondere mindestens 75 %, bevorzugt mindestens 80 %, ganz besonders bevorzugt mindestens 85 %, bezogen auf die spezifische Gesamtoberfläche (BET) der Aktivkohle. Insbesondere liegt dieser Mikroporenoberflächenanteil im Bereich von 70 bis 95 %, insbesondere 75 bis 95 %, vorzugsweise 80 bis 90 %.

Die erfindungsgemäß bevorzugt eingesetzte Aktivkohle weist aufgrund ihrer Mikroporosität gleichermaßen eine große Mikroporenoberfläche auf. Insbesondere liegt die Mikroporenoberfläche nach Carbon Black (d. h. die aus Poren mit Porendurchmessern von s 25 Å, vorzugsweise ≤ 20 Å, gebildete Mikroporenoberfläche) bei mindestens 400 m²/g, insbesondere mindestens 800 m²/g, vorzugsweise mindestens 1.000 m²/g, besonders bevorzugt mindestens 1.200 m²/g. Gemäß einer bevorzugten Ausführungsform liegt diese Mikroporenoberfläche im Bereich von 400 bis 1.750 m²/g, insbesondere 800 bis 1.500 m²/g, vorzugsweise 1.000 bis 1.400 m²/g, besonders bevorzugt 1.100 bis 1.300 m²/g.

Erfindungsgemäß bevorzugt wird als Aktivkohle eine mikroporöse Aktivkohle mit einem mittleren Porendurchmesser (Durchschnittsporendurchmesser) von höchstens 35 Å, vorzugsweise höchstens 30 Å, besonders bevorzugt höchstens 25 Å, eingesetzt. Insbesondere liegt dieser mittlere Porendurchmesser im Bereich von 15 bis 35 Å, insbesondere 15 bis 30 Å, vorzugsweise 15 bis 25 Å.

Was die Dichte der erfindungsgemäß bevorzugt eingesetzten Aktivkohle anbelangt, so liegt die Rohdichte der eingesetzten Aktivkohle im allgemeinen im Bereich von 700 bis 975 g/cm³, insbesondere 750 bis 950 g/cm³, vorzugsweise 800 bis 900 g/cm³. Die Schüttdichte der eingesetzten Aktivkohle liegt dagegen im Bereich von 300 bis 900 g/cm³, insbesondere 350 bis 800 g/cm³, vorzugsweise 400 bis 750 g/cm³.

Für eine besonders gute Effizienz ist es von Vorteil, wenn die eingesetzte Aktivkohle eine Gesamtporosität von 40 bis 70 %, insbesondere 45 bis 65 %, vorzugsweise 50 bis 60 %, aufweist.

Erfindungsgemäß bevorzugt wird als Aktivkohle eine Aktivkohle mit einem spezifischen Gesamtporenvolumen im Bereich von 0,1 bis 2,5 cm³/g, insbesondere 0,2 bis 2,0 cm³/g, vorzugsweise 0,3 bis 1,5 cm³/g, besonders bevorzugt 0,4 bis 1,0 cm³/g, eingesetzt. Dabei liegt der Anteil an Poren mit Porendurchmessern von s 36 Å bei mindestens 65 %, insbesondere mindestens 70 %, vorzugsweise mindestens 75 %, und kann Werte bis zu 95 %, insbesondere bis zu 90 %, erreichen.

Eine erfindungsgemäß besonders geeignete Aktivkohle, welche die vorgenannten Eigenschaften und Spezifikationen erfüllt, wird beispielsweise von der Blücher GmbH, Erkrath, Deutschland, oder von der AdsorTech GmbH, Premnitz, Deutschland, vertrieben.

Zur Steigung der Adsorptionsleistung kann die erfindungsgemäß eingesetzte Aktivkohle mit einer Imprägnierung versehen werden. Dies ist dem Fachmann als solches bekannt.

Eine derartige Imprägnierung kann beispielsweise ausgewählt sein aus der Gruppe von
(i) Metallen, vorzugsweise Kupfer, Silber, Cadmium, Platin, Palladium, Rhodium, Zink, Quecksilber, Titan, Zirkonium, Molybdän und/oder Aluminium, insbesondere deren Ionen und/oder Salzen;
(ii) Enzymen;
(iii) basischen Verbindungen, insbesondere organischen Basen, wie organischen Aminen;
(iv) sauren Verbindungen, insbesondere salz- und schwefelsauren Verbindungen oder freien organischen oder anorganischen Säuren;
sowie Mischungen der vorgenannten Imprägnierungen.

Für den Fall, daß eine mit einer Imprägnierung versehene Aktivkohle verwendet wird, wird die Imprägnierung im allgemeinen in einer Menge, bezogen auf die Aktivkohle, von 0,01 bis 30 Gew.-%, vorzugsweise 0,1 bis 20 Gew.-%, besonders bevorzugt 1 bis 15 Gew.-%, ganz besonders bevorzugt 1 bis 10 Gew.-%, auf die Aktivkohle aufgebracht.

Die vorgenannten Imprägnierverbindungen ermöglichen zum Teil die katalytische Zersetzung bestimmter Geruchs- und/oder Schadstoffe bzw. deren beschleunigten Abbau bzw. deren beschleunigte Adsorption.

Gegenstand der vorliegenden Erfindung - gemäß einem **zweiten** Aspekt der vorliegenden Erfindung - ist die Verwendung des zuvor beschriebenen erfindungsgemäßen Baumaterials zur Beseitigung von Schad- und/oder Geruchsstoffen bzw. zur Beseitigung von Emissionen von Schad- und/oder Geruchsstoffen, insbesondere mittels Adsorption, vorzugsweise in Gebäuden.

Die Beseitigung der Emissionen von Schad- und/oder Geruchsstoffen kann zu Zwecken der Entfernung von Schad- und/oder Geruchsstoffen in schad- und/oder geruchsstoffbelasteten Räumen, zu Zwecken der Sanierung schad- und/oder geruchsstoffbelasteter Räume oder zu Zwecken der Raumluftverbesserung bzw. zur Verbesserung des Raumklimas erfolgen. Sie kann aber auch zu Zwecken des Bautenschutzes erfolgen, insbesondere zu Verhinderung der Kontaminierung eines Bauwerks mit in die Raumluft abgegebenen Schad- und/oder Giftstoffen (z. B. bei chemischen Reinigungsbetrieben, chemischen Anlagen, Laboratorien etc.).

Im Rahmen der erfindungsgemäßen Verwendung wird für den Fall der Entfernung von Schad- und/oder Geruchsstoffen in schad- und/oder geruchsstoffbelasteten Räumen, insbesondere zu Zwecken der Sanierung, die schad- und/oder geruchsstofffreisetzende Emissionsquelle mit dem erfindungsgemäßen plattenförmigen Werkstoff bzw. mit der erfindungsgemäßen Bauplatte, vorzugsweise mit einer mit Aktivkohle versehenen und/oder beaufschlagten Oberfläche des plattenförmigen Werkstoffs bzw. der Bauplatte, abgedeckt und/oder bedeckt.

Für den Fall, daß das erfindungsgemäße Baumaterial zur Verbesserung der Raumluftverbesserung, beispielsweise zur Entfernung von Schad- und/oder Geruchsstoffen aus der Raumluft (z. B. Tabakrauch), oder aber zu Zwecken des Bautenschutzes eingesetzt wird, wird eine mit Aktivkohle versehene und/oder beaufschlagte Oberfläche des plattenförmigen Werkstoffs der Raumseite zugewandt.

Gleichermaßen ist es möglich für den Fall, daß beide Oberflächen des plattenförmigen Werkstoffs mit Aktivkohle beaufschlagt bzw. versehen sind, daß gleichzeitig die Emissionsquelle von Schad- und/oder Geruchsstoffen einerseits bedeckt ist und andererseits auch eine Oberfläche des plattenförmigen Werkstoffs der Raumseite zugewandt ist.

Im Ergebnis ermöglicht die vorliegende Erfindung somit eine effiziente Beseitigung von Schad- und/oder Geruchsstoffen in Gebäuden bzw. Innenräumen. Die erfindungsgemäßen, für den Trockenbau geeigneten Plattenmaterialien, beispielsweise Gipsplatten, werden mit Adsorbentien auf Aktivkohlebasis versehen, um eine gute Schutzwirkung zu erreichen. So können beispielsweise Giftstoffe, die aus der Wand in den Raum entweichen (z. B. PCB, PCP, Formaldehyd etc.) wirksam entfernt werden. Auch Giftstoffe, die aus dem Raum in das Gemäuer eindringen können (Stichwort: "Bautenschutz"), wie z. B. Isocyanate, können wirksam entfernt werden. Zusätzlich kann durch die Filtrationswirkung der eingesetzten Aktivkohle eine Raumluftverbesserung bzw. eine Verbesserung des Raumklimas erreicht werden.

Weitere Ausgestaltungen, Abwandlungen und Variationen der vorliegenden Erfindung sind für den Fachmann beim Lesen der Beschreibung ohne weiteres erkennbar und realisierbar, ohne daß er dabei den Rahmen der vorliegenden Erfindung verläßt.

Die vorliegende Erfindung wird nachfolgend anhand von Ausführungsbeispielen veranschaulicht, welche die vorliegende Erfindung jedoch keinesfalls beschränken.

### Ausführungsbeispiele:

### Ausführungsbeispiel 1:

Die Innenwände eines Fertigelementgebäudes, die durch langjährige Beaufschlagung mit PCB-belasteter Raumluft kontaminiert waren, wurden vollständig mit erfindungsgemäßen Gipsbauplatten beaufschlagt, auf deren zur Wand weisenden Oberfläche Aktivkohlekügelchen (mittlerer Durchmesser: 0,8 mm) in einer Auflagemenge von ca. 200 g/m² bei der Herstellung der Gipsplatten eingedrückt waren (freie Oberfläche der Aktivkohlekügelchen: mindestens 50 %, BET-Oberfläche: ca. 1.225 m²/g, Adsorptionsvolumen V_{ads}: ca. 700 cm³/g; Mikroporenvolumenanteil ≥ 65 %; Gesamtporenvolumen nach Gurvich ≥ 0,70 cm³/g; spezifischer Mikroporenoberflächenanteil ≥ 85 %; Mikroporenoberfläche nach Carbon Black: ca. 1.250 m²/g; mittlerer Porendurchmesser: < 25 Å; Gesamtporosität: ca. 55 %; Berstdruck pro Aktivkohlekugel: ca. 10 Newton).

Die mit Aktivkohle beaufschlagte Oberfläche der Bauplatten wurde zur Wandseite hin angeordnet. Nach Anbringung der erfindungsgemäßen Bauplatten sank die PCB-Raumluftkonzentration von ca. 11.500 ng/m³ auf unter 250 ng/m³ und blieb auch in der Folgezeit (Beobachtungszeitraum: 6 Monate) unter diesem Wert.

### Ausführungsbeispiel 2:

Die zuvor beschriebenen Gipsbauplatten nach der vorliegenden Erfindung wurden in einem Raum mit Normalklima ausgelegt, wobei auf 1 m³ Raumvolumen ca. 1,2 m² Gipsplattenfläche kam.

Als Vergleichsgipsbauplatten dienten handelsübliche Gipsbauplatten (Fa. Knauf Gips KG) mit ca. 10 Gew.-% natürlichen Zeolithen (nativ, nicht entwässert) mit einer Korngröße von maximal 200 µm und einem d₅₀-Wert von ca. 40 µm (ebenfalls ca. 1,2 m² Gipsplattenfläche auf 1 m³ Raumvolumen).

In den jeweiligen Räumen wurde durch kontinuierliche Zuführung eines definierten Schadstoffgemisches die jeweilige Schadstoffkonzentration mit den Schadstoffen Formaldehyd und Benzol sowie mit einem Schadstoffgemisch aus Zigarettenrauch eingestellt. Die Luftwechselrate des jeweiligen Gemisches betrugt 0,5 Wechsel/Stunde, so daß das Schadstoffgemisch innerhalb von zwei Stunden komplett ausgetauscht wurde. In dem ausströmenden Schadstoffgemisch wurde der verbleibende Schadstoffwert als Gaskonzentration gemessen.

Durch den Kontakt mit den Bauplatten wurden die Schadstoffe teilweise abgebaut bzw. entfernt, und es stellte sich in dem System eine Schadstoffkonzentration vom Ausgangswert (Einströmungsgemisch) zum Gleichgewichtswert (ausströmendes Gemisch) ein.

Während bei den Vergleichbauplatten mit den Zeolithen der Formaldehydgehalt von ca. 600 µg/m³ auf ca. 100 µg/m³ und der Benzolgehalt von ca. 45 µg/m³ auf ca. 10 µg/m³ gesenkt werden konnte, konnte er bei den erfindungsgemäßen Bauplatten deutlich stärker gesenkt werden, und zwar im Falle von Formaldehyd von ca. 600 µg/m³ auf ca. 50 µg/m³ und im Fall von Benzol von ca. 45 µg/m³ auf ca. 4 µg/m³.

Bei dem eingeströmten Zigarettenrauch wurde in beiden Fällen eine wesentliche Reduzierung der Geruchsbelästigung durch den Kontakt des Rauches mit den Gipsbauplatten durch Olfaktometrie nachgewiesen, wobei im Fall der erfindungsgemäßen Bauplatten dieser Effekt stärker als im Fall der Vergleichsbauplatten war.

Die zuvor geschilderten Ergebnisse belegen, daß die Entfernung von Schad- und/oder Geruchsstoffen durch die erfindungsgemäß vorgesehene Aktivkohle gegenüber handelsüblichen Bauplatten mit Zeolithen deutlich gesteigert werden kann. Dies belegt die Überlegenheit der erfindungsgemäßen Bauplatten gegenüber Bauplatten des Standes der Technik.

### Ausführungsbeispiel 3:

Ausführungsbeispiel 2 wurde wiederholt, jedoch wurde in Abweichung zu der in Ausführungsbeispiel 2 anstelle der mikroporösen Aktivkohle eine Aktivkohle mit geringerem Mikroporenanteil und folglich höherem Meso- und Makroporenanteil (mittlerer Durchmesser der Aktivkohlekugeln: 0,8 mm; freie Oberfläche der Aktivkohlekügelchen: mindestens 50 %; BET-Oberfläche: ca. 1.200 m²/g; Adsorptionsvolumen V_{ads}: ca. 680 cm³/g; Mikroporenvolumenanteil < 40 %; spezifischer Mikroporenoberflächenanteil < 30 %; mittlerer Porendurchmesser: > 40 Å; Gesamtporosität: ca. 45 %).

Der Formaldehydgehalt konnte von ca. 600 µg/m³ auf ca. 75 µg/m³ und der Benzolgehalt von ca. 45 µg/m³ auf ca. 8 µg/m³ gesenkt werden. Die erfindungsgemäßen Bauplatten mit der meso-/makroporösen Aktivkohle sind somit gegenüber den zeolithhaltigen Bauplatten des Standes der Technik deutlich effizienter, aber weniger effizient als im Fall der Verwendung mikroporöser Aktivkohle - und dies bei im übrigen im wesentlichen gleichen Parametern mit Ausnahme der Mikroporosität. Durch die Auswahl einer Aktivkohle hoher Mikroporosität läßt sich überraschenderweise die Effizienz deutlich steigern.

Auch in bezug auf die meso-/makroporöse Aktivkohle wurde bei dem eingeströmten Zigarettenrauch eine wesentliche Reduzierung der Geruchsbelästigung durch Olfaktometrie nachgewiesen.

## Patentansprüche

1. Baumaterial für den Innenausbau auf Basis eines plattenförmigen Werkstoffs für den Trockenbau,
**dadurch gekennzeichnet,**
**daß** der plattenförmige Werkstoff Aktivkohle aufweist.

2. Baumaterial nach Anspruch 1, **dadurch gekennzeichnet, daß** das plattenförmige Werkstoff eine Bauplatte ist, insbesondere eine Bauplatte aus Gips, aus Gipskarton, aus Gipsfaserverbundstoffen, wie eine Gipsfaserplatte, aus Zementfaserverbundstoff, wie eine Faserzementplatte, aus Glasfasern, aus Kohlenstoffasern, aus Holz, aus Holzfasern, aus Kunststoff oder dergleichen sowie Kombination hiervon, bevorzugt eine Gipsbauplatte oder aber eine Gipskartonplatte, insbesondere eine Gipskartonplatte mit ein- oder beidseitiger Kartonageaußenbeschichtung und einer Gipskernschicht.

3. Baumaterial nach Anspruch 1 und/oder 2, **dadurch gekennzeichnet, daß** die Aktivkohle in den plattenförmigen Werkstoff bei dessen Herstellung eingearbeitet ist.

4. Baumaterial nach Anspruch 1 und/oder 2, **dadurch gekennzeichnet, daß** der plattenförmige Werkstoff an mindestens einer seiner beiden Oberflächen mit Aktivkohle versehen und/oder beaufschlagt ist, insbesondere wobei die Aktivkohle an der bzw. den Oberfläche(n) des plattenförmigen Werkstoffs fixiert ist, insbesondere mittels eines vorzugsweise punktförmig und/oder punktrasterförmig aufgetragenen Klebestoffs oder durch Einarbeiten, insbesondere Eindrücken, in die Oberfläche(n) des plattenförmigen Werkstoffs bei dessen Herstellung, und/oder insbesondere wobei die Fixierung der Aktivkohle derart ist, daß mindestens 25 %, insbesondere mindestens 30 %, vorzugsweise mindestens 40 %, besonders bevorzugt mindestens 50 %, der Oberfläche der Aktivkohle für zu adsorbierende Schad- und/oder Geruchsstoffe frei zugänglich ist.

5. Baumaterial nach Anspruch 1 und/oder 2, **dadurch gekennzeichnet, daß** die Aktivkohle an einem vorzugsweise flächigen Träger, insbesondere einem textilen oder papierenen Flächengebilde, vorzugsweise einem Papier, einem Karton, einer Pappe oder dergleichen, fixiert ist und der mit Aktivkohle versehene Träger mit dem plattenförmigen Werkstoff verbunden ist, insbesondere mittels Laminierung, Verkleben oder dergleichen.

6. Baumaterial nach einem oder mehreren der vorangehenden Ansprüche, **dadurch gekennzeichnet, daß** die Menge an Aktivkohle derart ausgelegt ist, daß die durch die Aktivkohle bereitgestellte Adsorptionskapazität ausreicht, um bei der Verwendung des plattenförmigen Werkstoffs aus der Umgebung stammende Emissionen von Schad- und/oder Geruchsstoffen dauerhaft adsorptiv zu binden und/oder zu entfernen und/oder daß der plattenförmige Werkstoff die Aktivkohle in einer Menge, insbesondere Beladungs- und/oder Auflagemenge, von insgesamt 20 bis 750 g/m², insbesondere 25 bis 500 g/m², vorzugsweise 30 bis 400 g/m², besonders bevorzugt 50 bis 300 g/m², enthält.

7. Baumaterial nach einem oder mehreren der Ansprüche 1 bis 9, **dadurch gekennzeichnet, daß** die Aktivkohle pulverförmig ist oder aber daß die Aktivkohle in Form von Aktivkohlefasern vorliegt oder aber daß die Aktivkohle kornförmig, vorzugsweise kugelförmig, ausgebildet ist, insbesondere wobei die Aktivkohlekörner, vorzugsweise Aktivkohlekugeln, mittlere Teilchendurchmesser im Bereich von 0,01 bis 2,0 mm, insbesondere 0,05 bis 1,0 mm, vorzugsweise 0,1 bis 1,0 mm, aufweisen und/oder insbesondere wobei die einzelnen Aktivkohlekörner, vorzugsweise Aktivkohlekugeln, einen mittleren Abstand zueinander aufweisen, der mindestens der Hälfte, insbesondere mindestens Dreiviertel, vorzugsweise mindestens dem Einfachen, besonders bevorzugt mindestens dem Eineinhalbfachen, des mittleren Teilchendurchmessers der Aktivkohlekömer, vorzugsweise Aktivkohlekugeln, entspricht.

8. Baumaterial nach einem oder mehreren der vorangehenden Ansprüche, **dadurch gekennzeichnet, daß** die Aktivkohle ein Mischung aus faserförmiger und kornförmiger, vorzugsweise kugelförmiger, Aktivkohle umfaßt.

9. Baumaterial nach einem oder mehreren der vorangehenden Ansprüche, **dadurch gekennzeichnet, daß** die Aktivkohle eine spezifische Oberfläche (BET) von mindestens 500 m²/g, insbesondere mindestens 750 m²/g, vorzugsweise mindestens 1.000 m²/g, besonders bevorzugt mindestens 1.200 m²/g, aufweist und/oder daß die Aktivkohle eine spezifische Oberfläche (BET) von 500 bis 2.500 m²/g, insbesondere 750 bis 2.250 m²/g, vorzugsweise 900 bis 2.000 m²/g, besonders bevorzugt 1.000 bis 1.750 m²/g, aufweist.

10. Baumaterial nach einem oder mehreren der vorangehenden Ansprüche, **dadurch gekennzeichnet, daß** die Aktivkohle eine Aktivkohle mit einem Adsorptionsvolumen V_{ads} von mindestens 250 cm³/g, insbesondere mindestens 300 cm³/g, vorzugsweise mindestens 350 cm³/g, besonders bevorzugt mindestens 400 cm³/g, ist und/oder daß die Aktivkohle eine Aktivkohle mit einem Adsorptionsvolumen V_{ads} von 250 bis 1.000 cm³/g, insbesondere 300 bis 900 cm³/g, vorzugsweise 350 bis 750 cm³/g, ist und/oder daß die Aktivkohle eine Aktivkohle mit einem Gesamtporenvolumen nach Gurvich von mindestens 0,50 cm³/g, insbesondere mindestens 0,55 cm³/g, vorzugsweise mindestens 0,60 cm³/g, besonders bevorzugt mindestens 0,65 cm³/g, ganz besonders bevorzugt mindestens 0,70 cm³/g, ist und/oder daß die Aktivkohle eine Aktivkohle mit einem Gesamtporenvolumen nach Gurvich von 0,50 bis 0,90 cm³/g, insbesondere 0,55 bis 0,85 cm³/g, vorzugsweise 0,60 bis 0,80 cm³/g, besonders bevorzugt 0,65 bis 0,80 cm³/g, ganz besonders bevorzugt 0,70 bis 0,75 cm³/g, ist.

11. Baumaterial nach einem oder mehreren der vorangehenden Ansprüche, **dadurch gekennzeichnet, daß** die Aktivkohle eine Aktivkohle mit einem großen Mikroporenvolumenanteil, bezogen auf das Gesamtporenvolumen der Aktivkohle, ist und/oder daß die Aktivkohle eine Aktivkohle mit einem Anteil des Mikroporenvolumens, bezogen auf das Gesamtporenvolumen der Aktivkohle, von mindestens 60 %, insbesondere mindestens 65 %, bevorzugt mindestens 70 %, ist und/oder daß die Aktivkohle eine Aktivkohle mit einem aus Poren mit Porendurchmessern von ≤ 25 Å, vorzugsweise ≤ 20 Å, gebildeten Mikroporenvolumenanteil von mindestens 60 %, insbesondere mindestens 65 %, bevorzugt mindestens 70 %, bezogen auf das Gesamtporenvolumen, ist.

12. Baumaterial nach einem oder mehreren der vorangehenden Ansprüche, **dadurch gekennzeichnet, daß** die Aktivkohle eine Aktivkohle mit einem Mikroporenvolumen, insbesondere einem aus Poren mit Porendurchmessern von ≤ 25 Å, vorzugsweise ≤ 20 Å, gebildeten Mikroporenvolumen, nach Carbon Black von mindestens 0,40 cm³/g, insbesondere mindestens 0,45 cm³/g, vorzugsweise mindestens 0,50 cm³/g, ist und/oder daß die Aktivkohle eine Aktivkohle mit einem spezifischen Mikroporenoberflächenanteil, insbesondere einem aus Poren mit Porendurchmessern von ≤ 25 Å, vorzugsweise ≤ 20 Å, gebildeten spezifischen Mikroporenoberflächenanteil, von mindestens 70 %, insbesondere mindestens 75 %, bevorzugt mindestens 80 %, ganz besonders mindestens 85 %, bezogen auf die spezifische Gesamtoberfläche (BET) der Aktivkohle, ist.

13. Baumaterial nach einem oder mehreren der vorangehenden Ansprüche, **dadurch gekennzeichnet, daß** die Aktivkohle eine Aktivkohle mit einer Mikroporenoberfläche nach Carbon Black, insbesondere einer aus Poren mit Porendurchmessern von ≤ 25 Å, vorzugsweise ≤ 20 Å, gebildete Mikroporenoberfläche, von mindestens 400 m²/g, insbesondere mindestens 800 m²/g, vorzugsweise mindestens 1.000 m²/g, besonders bevorzugt mindestens 1.200 m²/g, ist und/oder daß die Aktivkohle eine Aktivkohle mit einem mittleren Porendurchmesser (Durchschnittsporendurchmesser) von höchstens 35 Å, vorzugsweise höchstens 30 Å, besonders bevorzugt höchstens 25 Å, ist.

14. Baumaterial nach einem oder mehreren der vorangehenden Ansprüche, **dadurch gekennzeichnet, daß** die Aktivkohle eine Aktivkohle mit einer Rohdichte im Bereich von 700 bis 975 g/cm³, insbesondere 750 bis 950 g/cm³, vorzugsweise 800 bis 900 g/cm³, und/oder mit einer Schüttdichte der im Bereich von 300 bis 900 g/cm³, insbesondere 350 bis 800 g/cm³, vorzugsweise 400 bis 750 g/cm³ und/oder mit einer Gesamtporosität von 40 bis 70 %, insbesondere 45 bis 65 %, vorzugsweise 50 bis 60 %, ist.

15. Baumaterial nach einem oder mehreren der vorangehenden Ansprüche, **dadurch gekennzeichnet, daß** die Aktivkohle eine Aktivkohle mit einem spezifischen Gesamtporenvolumen im Bereich von 0,1 bis 2,5 cm³/g, insbesondere 0,2 bis 2,0 cm³/g, vorzugsweise 0,3 bis 1,5 cm³/g, besonders bevorzugt 0,4 bis 1,0 cm³/g, ist, insbesondere wobei der Anteil an Poren mit Porendurchmessern von ≤ 36 Å mindestens 65 %, insbesondere mindestens 70 %, vorzugsweise mindestens 75 %, und bis zu 95 %, insbesondere bis zu 90 %, beträgt.

16. Baumaterial nach einem oder mehreren der vorangehenden Ansprüche, **dadurch gekennzeichnet, daß** die Aktivkohle eine Imprägnierung aufweist, insbesondere wobei die Imprägnierung ausgewählt ist der Gruppe von (i) Metallen, vorzugsweise Kupfer, Silber, Cadmium, Platin, Palladium, Rhodium, Zink, Quecksilber, Titan, Zirkonium, Molybdän und/oder Aluminium, insbesondere deren Ionen und/oder Salzen; (ii) Enzymen; (iii) basischen Verbindungen, insbesondere organischen Basen, wie organischen Aminen; (iv) sauren Verbindungen, insbesondere salz- und schwefelsauren Verbindungen oder freien organischen oder anorganischen Säuren; sowie deren Mischungen und/oder Kombination und/oder insbesondere wobei die Imprägnierung, bezogen auf die Aktivkohle, in einer Menge von 0,01 bis 30 Gew.-%, vorzugsweise 0,1 bis 20 Gew.-%, besonders bevorzugt 1 bis 15 Gew.-%, ganz besonders bevorzugt 1 bis 10 Gew.-%, auf die Aktivkohle aufgebracht ist.

17. Verwendung eines Baumaterials nach einem oder mehreren der vorangehenden Ansprüche zur Beseitigung von Emissionen von Schad- und/oder Geruchsstoffen, insbesondere mittels Adsorption, insbesondere in Gebäuden, vorzugsweise zur Entfernung von Schad- und/oder Geruchsstoffen in schad- und/oder geruchsstoffbelasteten Räume oder zur Sanierung schad- und/oder geruchsstoffbelasteter Räume oder zur Raumluftverbesserung und/oder zur Verbesserung des Raumklimas.

18. Verwendung nach Anspruch 17, **dadurch gekennzeichnet, daß** eine Schad- und/oder Geruchsstoffe freisetzende Emissionsquelle mit dem plattenförmigen Werkstoff, vorzugsweise mit einer mit Aktivkohle versehenen und/oder beaufschlagten Oberfläche des plattenförmigen Werkstoffs, abgedeckt und/oder bedeckt wird und/oder daß eine mit Aktivkohle versehene und/oder beaufschlagte Oberfläche des plattenförmigen Werkstoffs der Raumseite zugewandt wird.
